# EUROPÄISCHE PATENTANMELDUNG

(11) **EP 0 916 314 A1**
(43) Veröffentlichungstag der Anmeldung: **19.05.1999**
(21) Anmeldenummer: 98115878.5
(22) Anmeldetag: 22.08.1998
(51) Int. Cl.: A61B 17/32

(54) **Gefässstanze**

(30) Priorität: 17.11.1997 DE 19750803
(71) Anmelder: Fehling, Ulrike, 63791 Karlstein (DE)
(72) Erfinder: Fehling, Guido, 63791 Karlstein (DE); Zepf, Christoph, 78589 Dürbheim (DE)
(74) Vertreter: Patentanwälte Westphal, Mussgnug & Partner

(57) **Zusammenfassung**

Es wird eine Gefäßstanze beschrieben, bei welcher ein Scherblatt (32) mit einem Ringmesser (35) koaxial zusammenwirkt. Das Ringmesser (35) und das Scherblatt (32) werden durch das Schaftteil (20) und das Schieberteil (21) eines Schiebeschaftes mittels eines außerhalb der Achse dieses Schiebeschaftes angeordneten Branchengriffes (11) betätigt.

## Beschreibung

Die Erfindung betrifft eine Gefäßstanze gemäß dem Oberbegriff des Anspruchs 1.

Gefäßstanzen dienen zum Stanzen einer Öffnung in die Wandung von Blutgefäßen. Insbesondere werden bei Bypass-Operationen Öffnungen in die Gefäßwand gestanzt, um das Bypass-Gefäß anzuschliessen. Häufigstes Anwendungsgebiet der Gefäßstanzen ist die herznahe Aorta.

Aus der DE-U-296 21 041 und der US-A-51 92 294 sind Gefäßstanzen bekannt mit einem Ringmesser und einem koaxial zu dem Ringmesser angeordneten Scherblatt, wobei das Scherblatt und das Ringmesser relativ zueinander axial bewegbar sind. Das Ringmesser und das Scherblatt sind am distalen Ende eines Schaftes des Instruments angebracht. Die Betätigung erfolgt durch eine am proximalen Ende des Schaftes angeordnete Griffmechanik mit zwei Griffbranchen. Die Griffbranchen sind unter einem Winkel zur Achse des Instrumentschaftes angeordnet.

Um eine Öffnung in die Gefäßwand zu stanzen, wird das Scherblatt durch einen kleinen Einschnitt in das Gefäßinnere geführt, so daß die Gefäßwand zwischen Scherblatt und Ringmesser angeordnet ist. Zum Stanzen wird das Scherblatt mittels der Betätigung durch das Ringmesser gezogen, wodurch die Gefäßwand gleichförmig ausgeschnitten wird. Eine im Hohlschaft angebrachte Feder führt das Scherblatt zurück in seine Ausgangslage und gibt dabei das Schnittgut frei.

Die Ausbildung der Gefäßstanze mit einem Schaft und die Anordnung der Griffbranchen unter einem Winkel zu diesem Schaft machen die Gefäßstanze insbesondere für den Einsatz in tieferliegenden und schlecht zugänglichen Operationsgebieten, wie z. B. an der hernahen Aorta geeignet. Die Ausbildung der Griffbranchen und ihrer Anordnung in Bezug auf die Achse des Schaftes bewirken eine günstige Hebelübersetzung und eine geringe Behinderung der Sicht des Operateurs.

Bei diesen bekannten Gefäßstanzen ist der Schaft als Rohrschaft ausgebildet, bei welchem ein feststehendes Schaftrohr und eine axial in diesem verschiebbare Bestätigungsstange vorgesehen sind.

Bei der Gefäßstanze der DE-U-296 21 041 ist das Ringmesser als Schneide an dem distalen Ende des Schaftrohres ausgebildet. Das Scherblatt ist an der koaxial in dem Schaft geführten Betätigungssstange angeordnet. Daraus ergibt sich, daß bei dem Stanzvorgang das sich im Inneren des Gefäßes befindliche Scherblatt axial in das feststehende außerhalb des Gefäßes befindliche Ringmesser gezogen wird. Dies hat den Nachteil, daß bei der ohnehin eingeschränkten Einsehbarkeit das Scherblatt unbeabsichtigt durch den Einschnitt der Gefäßwand aus dem Gefäß herausgezogen werden kann.

Gemäß der US-A-5 192 294 wird dieser Nachteil vermieden. Das Scherblatt ist mittels eines axialen Stiftes und eines Querstiftes axial in dem distalen Ende des äußeren Schaftrohres gehalten. Das Ringmesser ist an einer Buchse ausgebildet, die in das distale Ende der axial verschiebbaren Betätigungsstange eingesetzt und von dem Querstift durchsetzt ist. Dadurch ergibt sich ein äußerst komplizierter Aufbau des Instruments. Das Zerlegen und Zusammensetzen der Gefäßstanze für die Reinigung und Sterilisation wird dadurch extrem aufwendig und erfordert eine hohe Geschicklichkeit.

Der Erfindung liegt die Aufgabe zugrunde, eine Gefäßstanze zur Verfügung zu stellen, die im Bezug auf Zerlegbarkeit und Handhabung verbessert ist.

Diese Aufgabe wird erfindungsgemäß gelöst durch eine Gefäßstanze mit den Merkmalen des Anspruchs 1.

Vorteilhafte Ausführungen und Weiterbildungen der Erfindung sind in den Unteransprüchen angegeben.

Die erfindungsgemäße Gefäßstanze ist als Schiebeschaftinstrument ausgebildet, wobei das Ringmesser und das Scherblatt mittels des feststehenden Schaftteiles und des axial verschiebbaren Schieberteiles betätigbar sind. Das Schaftteil und das Schieberteil werden durch zwei verschwenkbare Griffbranchen betätigt, die unter einem Winkel an dem Schiebeschaft angebracht sind.

Die Ausbildung der Gefäßstanze als Schiebeschaftinstrument ermöglicht es, den Schaft der Gefäßstanze lang und mit geringem Durchmesser herzustellen. Dadurch eignet sich die Gefäßstanze insbesondere auch für den Einsatz in tieferliegenden und schlecht zugänglichen Operationsgebieten, wie z. B. an der herznahen Aorta. Ebenso eignet sich die Gefäßstanze wegen der Ausbildung als Schiebeschaftinstrument mit schlankem langem Schaft auch für den Einsatz in der minimal-invasiven Chirurgie.

Die Ausbildung als Schiebeschaftinstrument ermöglicht ein einfaches Zerlegen des Instrumentes, wobei insbesondere die Zerlegung des Schaftes in Schaftteil und Schieberteil eine zuverlässige Reinigung und Sterilisierung ermöglicht, da keine rohrförmigen Hohlteile vorhanden sind. Die Gefäßstanze kann in einfacher Weise mit wenigen Handgriffen zerlegt und wieder montiert werden. Dies ist äußerst zeitsparend und erfordert wenig manuelle Geschicklichkeit. Die Gefäßstanze wird in nur wenige Einzelteile zerlegt, was nicht nur die Zerlegung und Montage erleichtert, sondern auch vermeidet, daß Einzelteile verloren gehen. Die Anordnungen der Griffbranchen zur Betätigung der Gefäßstanze außerhalb der Schaftachse und am proximalen Instrumentenende hat zur Folge, daß die das Gerät bedienende Hand des Operateurs die Sicht auf das Operationsfeld nicht oder nur minimal behindert. Außerdem bedingen die Griffbranchen eine günstige Hebelübersetzung und damit eine hohe Scherkraft beim Stanzvorgang. Auch häufig infolge von Verkalkung schwer zu durchtrennende Gefäßwände können ohne größeren Kraftaufwand der den Griff betätigenden Hand gestanzt werden. Dadurch ist ein feinfühliger Einsatz der Gefäßstanze und eine sensible Wahrnehmung des Gewebewiderstands beim Durchtrennen begünstigt.

Die Anbringung des Scherblattes an dem feststehenden Schaftteil und die Anbringung des Ringmessers an dem axial verschiebbaren Schieberteil haben außerdem zur Folge, daß das durch den Einschnitt der Gefäßwand in das Gefäßinnere eingesetzte Scherblatt beim Stanzvorgang festgehalten bleibt und das Ringmesser koaxial über das feststehende Scherblatt geschoben wird. Es ist dadurch vermieden, daß das Scherblatt unbeabsichtigt aus dem Gefäßinneren herausgezogen wird und der Stanzvorgang mißlingt.

Vorzugsweise wird auf die gesamte Gefäßstanze eine keramische Oberflächenbeschichtung physikalisch aufgedampft. Dadurch ergibt sich eine Oberflächenhärte mit reduziertem Anrieb und verringertem Reibungswiderstand. Die Oxydationsbeständigkeit ist verbessert und bei der Verwendung der Gefäßstanze störende Reflexionen sind vermieden. Die Standzeit der Funktionsteile, nämlich von Ringmesser und Scherblatt ist um ein vielfaches verlängert.

Im folgenden wird die Erfindung anhand eines in der Zeichnung dargestellen Ausführungsbeispieles näher erläutert. Es zeigen
- Figur 1: eine Seitenansicht der Gefäßstanze,

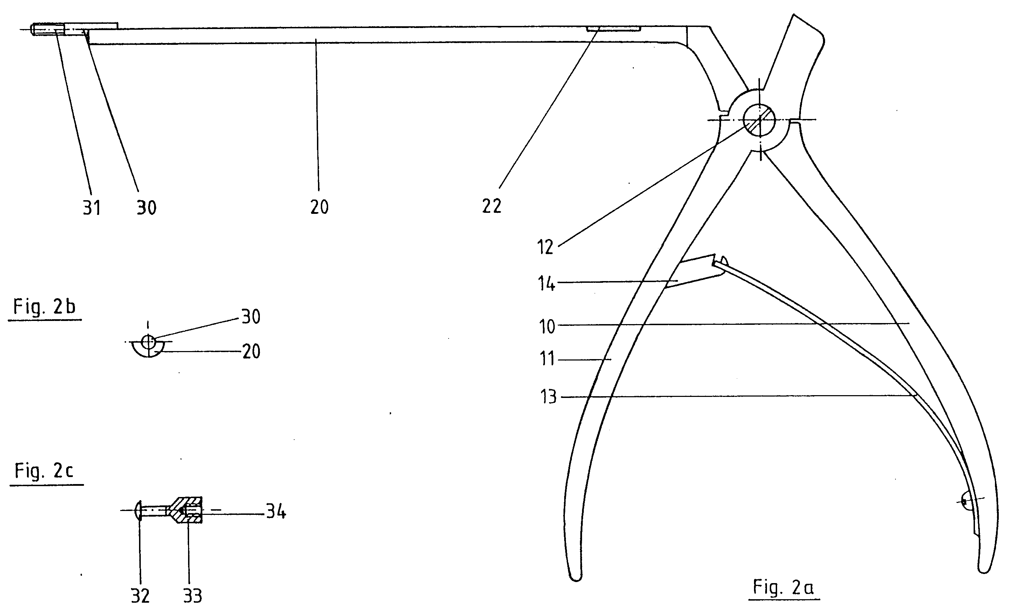

- Figur 2a: eine entsprechende Seitenansicht im demontierten Zustand mit abgenommenem Schieberteil
- Figur 2b: eine axiale Frontansicht des Schaftteiles der Figur 2a
- Figur 2c: das von dem Schaftteil demontierte Scherblatt,
- Figur3a: eine Seitenansicht des demontierten Schieberteiles,

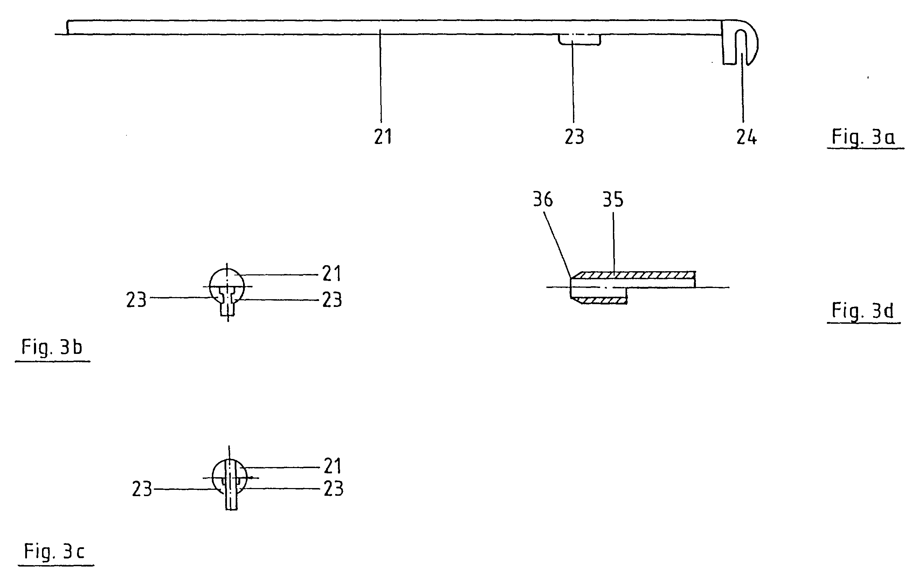

- Figur 3b: eine axiale Frontansicht des Schieberteiles der Figur 3a,
- Figur 3c: eine axiale Rückansicht des Schieberteiles der Figur 3a und
- Figur 3d: das Ringmesser in einem Axialschnitt.

Die Gefäßstanze weist einen Branchengriff auf, der aus einer feststehenden Griffbranche 10 und einer schwenkbaren Griffbranche 11 besteht, die durch einen Gelenkzapfen 12 schwenkbar miteinander verbunden sind. An der Innenseite der festen Griffbranche 10 ist eine als Blattfeder ausgebildete Spannfeder 13 mit ihrem einen Ende befestigt. Das freie andere Ende der Spannfeder 13 ist lösbar in eine Federaufnahme 14 eingehängt, die an der schwenkbaren Griffbranche 11 angebracht ist.

An der festen Griffbranche 10 ist einstückig das Schaftteil 20 eines Schiebeschaftes angeformt. An dem Schaftteil 20 ist axial verschiebbar das Schieberteil 21 des Schiebeschaftes geführt. Das Schaftteil 20 und das Schieberteil 21 weisen jeweils im wesentlichen einen halbkreisförmigen Querschnitt auf und liegen mit ihren Flachseiten aneinander an, so daß sie sich zu einem im wesentlichen kreisförmigen Gesamtquerschnitt des Schiebeschaftes ergänzen. Zur Führung des Schieberteiles 21 an dem Schaftteil 20 weist das Schaftteil 20 in seinem proximalen Bereich seitliche achsparallel verlaufende Führungsstege 22 auf, die von Führungslappen 23 des Schieberteiles 21 umgriffen werden. Um die Schwenkbewegung der schwenkbaren Griffbranche 11 in eine axiale Schubbewegung des Schieberteiles 21 umzusetzen, greift das Schieberteil 21 mit einem an seinem proximalen Ende ausgebildeten Aufnahmeschlitz 24 in einen Einschnitt der schwenkbaren Griffbranche 11 und umfaßt einen in diesem Einschnitt angeordneten Querstift.

Am distalen Ende des Schaftteiles 20 ist einstückig ein Stift 30 angebracht, der in der Achse des von Schaftteil 20 und Schieberteil 21 gebildeten kreisförmigen Querschnittes des Schiebeschaftes verläuft und sich distal nach vorn über das Schaftteil 20 erstreckt. An dem distal vorderen Ende des Stiftes 30 ist ein Gewinde 31 angebracht. Ein kreisscheibenförmiges Scherblatt 32 ist am vorderen Ende eines Stabes 33 angebracht, der mit einer Gewindebohrung seines verdickten hinteren Endes auf das Gewinde 31 des Stiftes 30 aufschraubbar ist. Dadurch ist das Scherblatt 32 demontierbar an dem Stift 30 und damit an dem Schaftteil 20 befestigt.

Am distalen Ende des Schieberteiles 21 ist ein Ringmesser 35 befestigt. Das Ringmesser 35 besteht aus einer zylindrischen Hülse, deren lichter Innendurchmesser dem Außendurchmesser des Scherblattes 32 und des hinteren Endes des Stabes 33 entspricht. Die distale Vorderkante der Hülse ist als ringförmige Schneidkante 36 ausgebildet, die mit der Umfangskante des Scherblattes 32 für den Stanzvorgang zusammenwirkt. Der Außendurchmesser der Hülse des Ringmessers 35 entspricht dem Außendurchmesser des durch Schaftteil 20 und Schieberteil 21 gebildeten Schiebeschaftes. Im axial hinteren Bereich ist die Hülse des Ringmessers 35 axial halbiert und ist mit ihrem proximal hinteren Ende an dem distal vorderen Ende des Schieberteiles 21 befestigt vorzugsweise durch Laser-Schweißen.

Das Ringmesser 35 umschließt koaxial den Stab 33 des Scherblattes 32 vor dem distalen Ende des Schaftteiles 20 und wird durch das verdickte hintere Ende des Stabes 33 geführt.

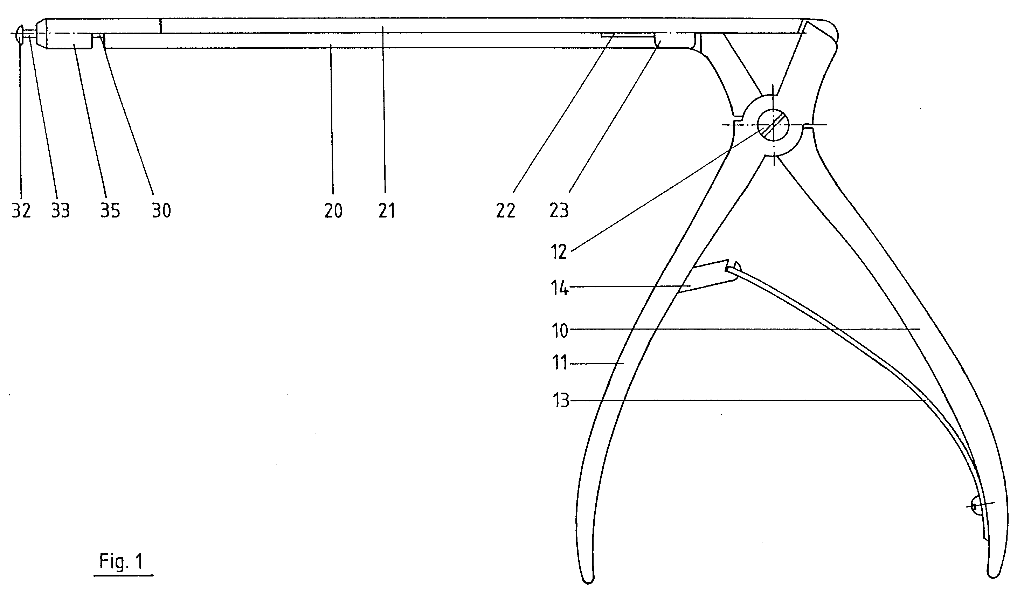

In der in Figur 1 gezeigten Grundstellung hält die Spannfeder 13 die Griffbranchen 10 und 11 in der gespreizten Stellung. Die schwenkbare Griffbranche 11 zieht das Schieberteil 21 nach hinten, so daß das mit dem Schieberteil 21 verbundene Ringmesser 35 zurückgezogen wird und das Scherblatt 32 distal aus dem Ringmesser 35 heraus ragt. Um ein Loch in eine Gefäßwand zu stanzen, wird ein kleiner Einschnitt in der Gefäßwand vorgenommen. Durch diesen Einschnitt wird das Scherblatt 32 in das Innere des Gefäßes eingesetzt, wobei die Gefäßstanze die in Figur 1 gezeigte Stellung einnimmt. Ist die Gefäßwand zwischen der Schneidkante 36 des Ringmessers 35 und dem Scherblatt 32 positioniert, werden die Griffbranchen 10 und 11 zusammengedrückt, wodurch das Schieberteil 21 vorgeschoben wird und sich das Ringmesser 35 koaxial über das Scherblatt 32 schiebt. Dabei wirken die Schneidkanten 36 des Ringmessers 35 mit der Umfangsschneidkante des Scherblattes 32 zusammen und stanzen einen dem Durchmesser des Scherblattes 32 entsprechenden Teil der Gefäßwand aus. Wird die Gefäßstanze aus dem Operationsfeld herausgezogen und die Griffbranchen 10 und 11 freigegeben, so spreizt die Spannfeder 13 die Griffbranchen 10 und 11 wieder auseinander, das Ringmesser 35 wird wieder in die in Figur 1 gezeigte Stellung zurückgezogen und das ausgestanzte Schnittgut kann entfernt werden.

Um die Gefäßstanze zu reinigen und zu sterilisieren, wird die Gefäßstanze in folgender Weise zerlegt: Zunächst wird die Spannfeder 13 aus der Federaufnahme 14 herausgedrückt, so daß die schwenkbare Griffbranche 11 von der festen Griffbranche 10 entriegelt ist. Die schwenkbare Griffbranche 11 kann nun über die in Figur 1 gezeigte Stellung hinaus von der festen Griffbranche 10 abgespreizt werden, wodurch das Schieberteil 21 auf dem Schaftteil 20 so weit nach hinten gezogen wird, daß die Führungslappen 23 außer Eingriff von den Führungsstegen 22 kommen. Das Schieberteil 21 kann nun von dem Schaftteil 20 nach oben abgehoben werden, wobei der Aufnahmeschlitz 24 des Schieberteiles 21 von der schwenkbaren Griffbranche 11 außer Eingriff kommt. Das nun freie Schieberteil 21 wird distal nach vorn geschoben, so daß das Ringmesser 35 axial über das Scherblatt 32 geschoben wird und damit auch das distale Ende des Schieberteiles 21 frei ist. Die Gefäßstanze ist somit in zwei Teile zerlegt, die einfach gereinigt und sterilisiert werden können.

Die Montage der Gefäßstanze ist in ebenso einfacher Weise in umgekehrter Reihenfolge möglich. Zunächst wird das Ringmesser 35 über das Scherblatt 32 gezogen, dann wird das Schieberteil 21 mit dem Aufnahmeschlitz 24 in die schwenkbare Griffbranche 11 eingehängt. Nun werden die Griffbranchen 10 und 11 in die in Figur 1 gezeigte Stellung geschlossen, so daß die Führungslappen 23 des Schieberteiles 21 mit den Führungsstegen 22 des Schaftteiles 20 in Eingriff kommen. Dann wird die Spannfeder 13 in die Federaufnahme 14 eingehängt, um den Griff in der in Figur 1 gezeigten Stellung zu verriegeln.

Die gesamte Gefäßstanze, insbesondere das Ringmesser 35 und das Scherblatt 32 sind aus gehärtetem rostfreiem Stahl gefertigt und mit einer keramischen Oberflächenbeschichtung versehen, die nach dem PVD-Verfahren (Physical-Vapor-Deposition) aufgedampft ist. Die wesentlichen Funktionsteile, nämlich das Scherblatt 32 und das Ringmesser 35 sind als Einzelersatzteile austauschbar, was die Wirtschaftlichkeit der Gefäßstanze erhöht.

### Bezugszeichenliste

- 10: feste Griffbranche
- 11: schwenkb. Griffbranche
- 12: Gelenkzapfen
- 13: Spannfeder
- 14: Federaufnahme
- 20: Schaftteil
- 21: Schieberteil
- 22: Führungsstege
- 23: Führungslappen
- 24: Aufnahmeschlitz
- 30: Stift
- 31: Gewinde
- 32: Scherblatt
- 33: Stab
- 34: Gewindebohrung
- 35: Ringmesser
- 36: Schneidkante

## Patentansprüche

1. Gefäßstanze, mit einem Schaft (20, 21), an dessen distalem Ende ein Ringmesser (35) und koaxial zu dem Ringmesser (35) ein Scherblatt (32) angeordnet sind, mit einer Griffmechanik, die zwei gegeneinander verschwenkbare Griffbranchen (10, 11) aufweist, die an dem proximalen Ende des Schaftes (20, 21) unter einem Winkel in bezug zu der Achse des Schaftes (20, 21) angeordnet sind, wobei das Scherblatt (32) und das Ringmesser (35) mittels der Griffmechanik relativ zueinander axial bewegbar sind, dadurch gekennzeichnet, daß der Schaft als Schiebeschaft mit einem mit der feststehenden Griffbranche (10) verbundenen Schaftteil (20) und einem an dem Schaftteil (20) geführten und mittels der schwenkbaren Griffbranche (11) verschiebbaren Schieberteil (21) ausgebildet ist, daß das Scherblatt (32) an einem in der Achse des Schaftes verlaufenden, am distalen Ende des Schaftteiles (20) angebrachten Stift (30) befestigt ist und daß das Ringmesser (35) an dem distalen Ende des Schieberteiles (21) befestigt ist und den das Scherblatt (32) tragenden Stift (30) koaxial vor dem distalen Ende des Schaftteiles (20) umschließt.

2. Gefäßstanze nach Anspruch 1, dadurch gekennzeichnet, daß das Scherblatt (32) mittels eines Gewindes (31, 34) auswechselbar an dem Stift (30) befestigt ist.

3. Gefäßstanze nach Anspruch 1 oder 2, dadurch gekennzeichnet, daß die schwenkbare Griffbranche (11) mit dem Schieberteil (21) kuppelbar ist, daß die Griffbranchen (10, 11) in einer maximalen Spreizstellung verriegelbar sind und daß nach Entriegelung die eine Griffbranche (11) über diese maximale Spreizstellung hinaus geschwenkt werden kann, wodurch das Schieberteil (21) aus der Führung (22, 23) des Schaftteiles (20) freikommt.

4. Gefäßstanze nach einem der Ansprüche 1 bis 3, dadurch gekennzeichnet, daß zumindest das Ringmesser (35) und das Scherblatt (32), vorzugsweise auch das Schaftteil (20) und das Schieberteil (21) mit einer keramischen Oberflächenbeschichtung versehen sind.
